# FASCICULE DE BREVET EUROPEEN

(11) **EP 0 897 309 B1**
(45) Date de publication et mention de la délivrance du brevet: **07.08.2002**
(21) Numéro de dépôt: 97923138.8
(22) Date de dépôt: 06.05.1997
(51) Int. Cl.: A61M 25/10, A61M 25/00

(54) **CATHETER DESTINE NOTAMMENT A LA DELIVRANCE LOCALE D'UNE SUBSTANCE THERAPEUTIQUEMENT ACTIVE**
KATHETER ZUR LOKALEN VERABREICHUNG EINER THERAPEUTISCH AKTIVEN SUBSTANZ
CATHETER IN PARTICULAR FOR THE DELIVERY OF A THERAPEUTICALLY ACTIVE SUBSTANCE

(30) Priorité: 06.05.1996 FR 9605615
(43) Date de publication de la demande: 24.02.1999
(73) Titulaire: CathNet-Science S.A., 75644 Paris Cedex 13 (FR)
(72) Inventeur: BOUSSIGNAC, Georges, F-92160 Antony (FR); LAGARDE, Vincent, F-75011 Paris (FR)
(74) Mandataire: Boydell, John Christopher
(86) Numéro de dépôt international: FR9700799
(87) Numéro de publication internationale: WO9741915

(56) Documents cités:
- EP-A- 0 526 102
- US-A- 5 304 132

## Description

La présente invention a pour objet un cathéter destiné à être introduit dans un conduit corporel où circule un fluide, notamment pour y délivrer, dans une zone localisée dudit conduit, une substance thérapeutiquement active, sans interrompre la circulation dudit fluide.

L'invention trouve principalement application dans le domaine du traitement de conduits sanguins et plus particulièrement encore dans le traitement de la resténose.

Le cathéter conforme à la présente invention peut cependant être utilisé pour le traitement d'affections variées de divers conduits morphologiques du corps humain ou animal comme en particulier les conduits urinaires et notamment l'urètre, ou bien encore les conduits digestifs et notamment l'oesophage.

Ce cathéter est principalement destiné à la délivrance locale d'une substance thérapeutiquement active mais permet également d'isoler un segment de la paroi d'un conduit corporel, par exemple pour aider une intervention chirurgicale sur ledit segment de paroi ainsi isolé.

Pour la clarté de l'exposé, le cathéter conforme à la présente invention sera décrit dans son application particulière préférée au traitement préventif de la resténose.

On sait que la resténose peut être définie comme une réduction récurrente du calibre de la lumière coronaire au site initialement dilaté avec succès lors d'une angioplastie transluminale coronaire.

Cette affection reste la principale limite actuelle de l'angioplastie coronaire par ballonnet puisqu'elle conduit à une nouvelle angioplastie dans environ 15 à 30 % des cas dans les 6 mois qui suivent une angioplastie réussie.

Diverses solutions ont été envisagées jusqu'à ce jour pour la prévention de la resténose qui ont conduit à des résultats plus ou moins satisfaisants.

Ainsi, l'utilisation de techniques d'angioplastie dites "ablatives" comme par exemple l'angioplastie par laser ou l'athérectomie n'a pas permis de réduire de façon significative le taux de resténose.

En revanche, la mise en place d'une endoprothèse coronaire après angioplastie constitue un traitement relativement efficace dans la prévention de la resténose, puisqu'elle conduit à une diminution d'environ 30 % du risque de resténose.

Cependant, la mise en place systématique d'endoprothèses ne peut être envisagée dans la mesure où ceci conduirait à appareiller inutilement un nombre important de patients qui n'auraient pas eu de resténose, sans endoprothèse, et où il subsisterait de toute façon un nombre relativement important de patients présentant une resténose malgré la mise en place d'une endoprothèse.

L'administration par voie systémique de substances pharmacologiquement actives telles que des anti-thrombines, des inhibiteurs de l'enzyme de conversion, certains vaso-dilatateurs ou certains agents hypo-lipidémiants n'a, à ce jour, pas conduit à des résultats entièrement satisfaisants.

C'est pourquoi, l'utilisation de dispositifs destinés à délivrer localement des substances thérapeutiquement actives constitue une perspective plus intéressante dans la prévention de la resténose.

En effet, cette méthode permet d'administrer, au niveau du site dilaté, des produits spécifiquement dirigés contre les mécanismes de la resténose, y compris des produits toxiques pour le reste de l'organisme, en des concentrations pré déterminées et pendant une durée variable.

Pour être utilisable dans le cadre de cette méthode, le dispositif doit permettre la délivrance locale de la substance active sans interruption de la circulation du fluide dans le conduit corporel.

Un cathéter répondant à cet objectif a été proposé dans le document EP 0.526.102.

Ce cathéter se compose généralement d'un élément tubulaire allongé comportant une structure gonflable disposée au niveau de sa partie distale.

Cette structure gonflable est constituée d'un ensemble de ballons toriques identiques et d'une membrane fixée à ces ballons, cette structure étant conformée de telle sorte qu'à l'état gonflé elle définit :
- d'une part, un conduit central de passage pour le fluide corporel et ;
- d'autre part une cavité, ou poche sensiblement étanche, destinée à contenir ladite substance thérapeutiquement active et délimitée par les ballons, par la surface externe de la membrane et par la surface interne du canal corporel.

Le corps de cathéter comporte un premier conduit interne communiquant d'une part avec les ballons et, d'autre part avec un moyen permettant le gonflage et le dégonflage de ces ballons ; et un second conduit interne communiquant d'une part avec la cavité précitée et, d'autre part avec une source d'alimentation en fluide contenant une substance pharmacologiquement active.

Comme on peut le voir dans le document précité, sur les schémas illustrant les différents modes de réalisation de ce cathéter, le corps de cathéter ainsi que les différents ballons intermédiaires destinés à maintenir la membrane en position sont en contact direct avec le conduit corporel.

Il en résulte qu'un tel dispositif ne permet pas de traiter efficacement, c'est-à-dire en une seule opération, toute la surface du conduit corporel au niveau du site dilaté, puisque la substance active ne peut atteindre la portion de surface du conduit corporel en contact avec les ballons et le corps de cathéter, sauf à déplacer la structure gonflable ce qui complique l'intervention.

La présente invention a pour but de résoudre ce problème technique en fournissant un cathéter d'une nouvelle conception, plus simple à réaliser et à mettre en oeuvre, et qui permette notamment la délivrance d'une substance thérapeutiquement active en une seule opération sur l'intégralité de la surface du conduit corporel à traiter.

La solution conforme à la présente invention, pour résoudre ce problème technique, consiste en un cathéter destiné à être introduit dans un conduit corporel où circule un fluide notamment pour y délivrer, dans une zone localisée, une substance thérapeutiquement active véhiculée par un second fluide, du type comprenant :
- un corps de cathéter comportant un élément tubulaire allongé présentant une partie proximale et une partie distale ;
- une structure gonflable disposée au niveau de la partie distale dudit corps de cathéter et comportant :
   - un premier élément gonflable et un deuxième élément gonflable qui, à l'état gonflé :
      - sont axialement espacés l'un de l'autre ;
      - portent chacun de façon étanche contre la paroi interne dudit conduit corporel ; et
      - présentent chacun une ouverture sensiblement centrale ;
   - une membrane fixée auxdits premier et deuxième éléments gonflables et définissant avec ces derniers, lorsque ceux-ci sont à l'état gonflé ;
      - d'une part, un manchon sensiblement cylindrique reliant entre elles de façon étanche les ouvertures centrales desdits premier et deuxième éléments gonflables formant ainsi un conduit de passage du fluide corporel ; et
      - d'autre part, une cavité sensiblement étanche susceptible de contenir ladite substance thérapeutiquement active et délimitée par lesdits premier et deuxième éléments gonflables, par la surface externe de ladite membrane et par la surface interne du conduit corporel ;
   - un troisième élément gonflable disposé entre lesdits premier et deuxième éléments gonflables conformé pour maintenir à l'état gonflé ladite membrane en position ;
ledit corps de cathéter comportant un premier conduit interne communiquant d'une part avec lesdits premier, deuxième et troisième éléments gonflables et d'autre part avec un moyen permettant le gonflage et le dégonflage de ces éléments ; et un second conduit interne communiquant d'une part avec la cavité précitée et d'autre part avec une source d'alimentation en fluide contenant ladite substance pharmacologiquement active, et éventuellement un troisième conduit interne, indépendant dudit second conduit interne, communiquant d'une part avec la cavité précitée et d'autre part avec une source d'alimentation en fluide contenant éventuellement une substance thérapeutiquement active ou avec un dispositif d'aspiration, ledit corps de cathéter étant disposé à l'intérieur de l'enroulement dudit tube, dans le conduit central précité caractérisé en ce que lesdits premier, deuxième et troisième éléments gonflables sont réalisés en une seule pièce préformée constituée d'un tube enroulé de façon hélicoïdale, ledit troisième élément étant constitué par une ou plusieurs spires de cet enroulement, le diamètre dudit tube au niveau de la portion constituant ledit troisième élément étant inférieur au diamètre dudit tube au niveau des portions de tube constituant respectivement lesdits premier et deuxième éléments et destinées à porter contre la paroi interne dudit conduit corporel.

Ainsi, comme on le comprend, l'originalité de la présente invention réside dans le fait que ni le corps de cathéter, ni le troisième élément gonflable destiné à maintenir la membrane en position ne sont directement au contact de la surface interne du conduit morphologique, ce qui permet de traiter, en une seule opération, l'intégralité de la surface du conduit corporel au niveau du site choisi.

Cet objectif est atteint d'une part dans la mesure où la structure gonflable entoure le corps de cathéter, de telle sorte que celui-ci n'est pas au contact du conduit corporel, et d'autre part dans la mesure où le tube constituant la structure gonflable présente un diamètre variable, les portions de tube délimitant la cavité destinée à contenir la substance active présentant un diamètre supérieur à celui de la portion de tube intermédiaire constituant la partie destinée à maintenir la membrane, de telle sorte que cette dernière portion du tube n'est pas au contact du conduit corporel.

Selon une caractéristique particulière de l'invention, la portion de tube constituant respectivement les premier et deuxième éléments gonflables présente une configuration en hélice à pas variable définissant une première zone constituée d'au moins deux spires jointives destinées à porter de façon étanche contre la paroi interne du conduit corporel, et une deuxième zone prolongeant la première et s'étendant jusqu'au corps de cathéter, ladite deuxième zone formant au moins une portion d'hélice dont le pas est supérieur à celui de l'hélice formée par les spires de la première zone, le diamètre du tube au niveau de la deuxième zone étant de préférence inférieur au diamètre du tube au niveau de la première zone.

Cette conformation des premier et deuxième éléments gonflables est particulièrement avantageuse puisqu'elle rend atraumatique la progression du cathéter lors de l'entrée et surtout du retrait de celui-ci dans le conduit corporel.

Pour faciliter le pliage et le repliage de la structure gonflable autour du corps de cathéter et rendre ainsi plus aisés l'introduction et le retrait de ce dernier, la structure gonflable est préformée de préférence de façon à présenter, vue en coupe transversale relativement au corps de cathéter, la forme générale d'une spirale refermée sur elle-même par une série d'au moins deux boucles alternées.

Selon une autre caractéristique particulière de l'invention, pour faciliter encore le repliage après une intervention, le cathéter précité est pourvu de moyens d'aide au repliage après dégonflage de la structure gonflable précitée.

Selon un premier mode de réalisation, ces moyens comprennent deux fils élastiques disposés respectivement autour des premier et deuxième éléments gonflables en y étant liés de préférence selon une génératrice.

Selon un autre mode de réalisation, ces moyens comprennent deux membranes élastiques sensiblement cylindriques enveloppant respectivement les premier et deuxième éléments gonflables en y étant liées de préférence selon une génératrice.

La conformation de la structure gonflable, associée aux moyens d'aide au repliage précités, garantit la sécurité du cathéter conforme à l'invention lors de son introduction et de son retrait du conduit corporel.

L'invention sera mieux comprise, et d'autres buts, caractéristiques et avantages de celle-ci apparaîtront plus clairement à la lecture de la description explicative qui va suivre, faite en référence aux dessins schématiques annexés donnés uniquement à titre d'exemple non limitatif illustrant un mode de réalisation actuellement préféré de l'invention, et dans lesquels :
- La figure 1 est une vue schématique longitudinale montrant l'extrémité distale d'un cathéter conforme à un mode de réalisation actuellement préféré de l'invention, représenté à l'état gonflé dans un conduit corporel ;
- La figure 2A est une vue en coupe transversale selon la ligne IIA-IIA de la figure 1 ;
- La figure 2B est une vue en coupe transversale selon la ligne IIB-IIB de la figure 1 ;
- La figure 2C est une vue en coupe transversale selon la ligne IIC-IIC de la figure 1 ;
- La figure 3 est une vue en coupe longitudinale illustrant l'extrémité distale d'un cathéter conforme à un autre mode de réalisation particulier de l'invention comportant des moyens d'aide au repliage de la structure gonflable, représenté à l'état gonflé .
- La figure 4 est une vue semblable à la figure 3 d'un même cathéter comportant d'autres moyens d'aide au repliage de la structure gonflable ;
- La figure 5 est une vue schématique en coupe transversale d'un cathéter conforme à la présente invention pourvu de moyens d'aide au repliage et représenté à l'état gonflé ;
- La figure 6 est une vue en coupe transversale du même cathéter, la structure gonflable étant à l'état dégonflé et replié ;
- La figure 7 est une vue schématique longitudinale montrant l'extrémité distale d'un cathéter conforme à un autre mode de réalisation de l'invention présentant un second et un troisième conduits internes;
- La figure 7A est une vue en coupe transversale selon la ligne A-A de la figure 7; et
- La figure 8 est une vue partielle du corps de cathéter suivant la vue F de la figure 7.

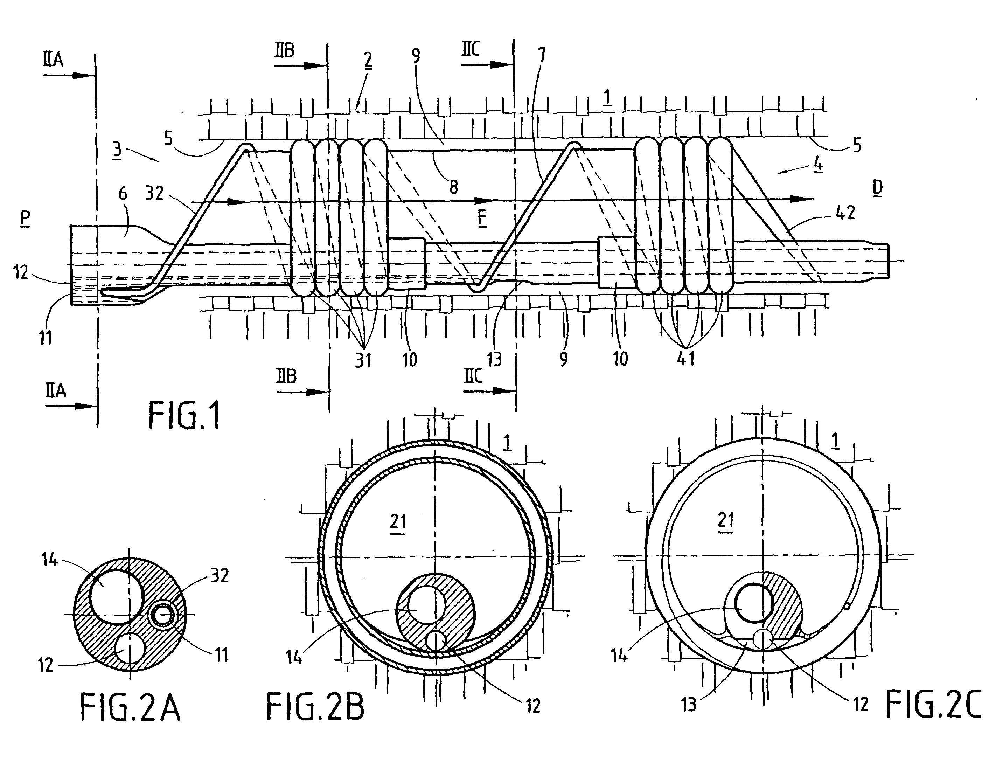

On a donc représenté à la figure 1 l'extrémité distale d'un cathéter équipé d'une structure gonflable conforme à un mode de réalisation actuellement préféré de l'invention, à l'état gonflé dans un conduit corporel tel que par exemple un vaisseau sanguin désigné par le chiffre de référence 1.

Un cathéter conforme à la présente invention comprend essentiellement un corps de cathéter comportant un élément tubulaire allongé présentant une partie proximale et une partie distale ainsi qu'une structure gonflable généralement désignée par le chiffre de référence 2 disposée au niveau de la partie distale du corps de cathéter.

Sur la figure 1, seule la partie de corps de cathéter portant la structure gonflable est représentée.

La partie proximale du cathéter non représentée et désignée par la lettre P est située à gauche sur la figure 1, tandis que la partie distale non représentée et désignée par la lettre D est située à droite.

La structure gonflable 2 comprend généralement un tube préformé de diamètre variable ayant une configuration en spires hélicoïdales définissant trois zones respectivement désignées par "premier", "deuxième" et "troisième" élément gonflable, ainsi qu'une membrane fixée à ce tube.

A l'état gonflé, représenté à la figure 1, le premier élément gonflable 3 et le deuxième élément gonflable 4 sont axialement espacés l'un de l'autre, portent chacun de façon étanche contre la paroi interne du conduit corporel 1 et présentent chacun une ouverture sensiblement centrale 21.

Comme on peut le voir, la portion de tube constituant respectivement les premier et deuxième éléments gonflables 3, 4 présente généralement une configuration en hélices à pas variable définissant une première zone constituée d'au moins deux spires jointives destinées à porter de façon étanche à l'état gonflé contre la paroi interne 5 du conduit corporel 1 et une deuxième zone prolongeant la première et s'étendant jusqu'au corps de cathéter 6, ladite deuxième zone formant au moins une portion d'hélice dont le pas est supérieur à celui de l'hélice formée par lesdites spires de la première zone, le diamètre du tube au niveau de la deuxième zone étant de préférence inférieur au diamètre du tube au niveau de la première zone.

Le nombre de spires jointives constituant la première zone des premier et deuxième éléments gonflables 3, 4 peut varier de 2 à plusieurs dizaines en fonction notamment du calibre du conduit corporel et du traitement à effectuer.

A titre d'exemple, la première zone des premier et deuxième éléments gonflables 3, 4 peut comprendre quatre spires jointives respectivement désignées par les chiffres de référence 31 et 41.

La distance entre le groupe de spires jointives proximales 31 et le groupe de spires jointives distales 41 peut varier dans de larges limites, par exemple entre 5mm et 100mm.

Cette distance sera d'environ 10mm dans le cas d'un traitement préventif de la resténose.

La deuxième zone des premier et deuxième éléments gonflables 3, 4 respectivement représentée par les chiffres de référence 32 et 42 forme au moins une portion d'hélice dont le pas est supérieur à celui de l'hélice formée dans la première zone. Généralement cette zone formera sensiblement une spire.

Les portions de tube formant les deuxièmes zones 32 et 42 s'étendent chacune jusqu'au corps de cathéter en donnant de préférence à la structure gonflable 2 vue en coupe transversale, la forme générale d'un trapèze dont la grande base est située au niveau du cathéter.

Avantageusement, les portions de tube 32 et 42 présentent un diamètre inférieur à celui des portions de tube définissant les spires jointives 31 et 41.

A titre d'exemple, le diamètre du tube au niveau des portions 32 et 42 peut être de l'ordre de 0.5mm et le diamètre du tube au niveau des spires jointives 31 et 41 de 0,7mm.

La portion de tube définissant le troisième élément gonflable 7 reliant entre eux les groupes de spires 31 et 41 présente également une configuration en hélice et peut comprendre une ou plusieurs spires selon la distance séparant les groupes de spires 31 et 41.

Le diamètre de la portion de tube définissant le troisième élément gonflable 7 est inférieur au diamètre du tube au niveau des spires 31 et 41.

A titre d'exemple, le diamètre du tube au niveau de l'élément gonflable 7 peut être de l'ordre de 0,5mm lorsque le diamètre du tube au niveau des spires 31 et 41 est de l'ordre de 0,7mm.

Ceci a notamment pour conséquence que le troisième élément gonflable 7 ne se trouve pas directement en contact avec la surface interne 5 du conduit corporel 1 comme on peut le voir sur la figure 1, ce qui constitue l'une des caractéristiques originales du cathéter conforme à l'invention.

Avantageusement, le pas de l'hélice au niveau du troisième élément 7 sera supérieur à celui de l'hélice formée par les groupes de spires 31 et 41.

Cependant, il ne s'agit là que d'un mode de réalisation préféré, les spires constituant le troisième élément gonflable pouvant être également jointives entre elles et éventuellement avec les spires 31 et 41 des premier et deuxième éléments gonflables.

Le tube formant les premier, deuxième et troisième éléments gonflables peut être avantageusement réalisé en une matière thermoplastique telle que par exemple un polyamide, un polyéthylène réticulé ou non, un polyuréthane ou un polyéthylène téréphtalate.

Ce tube peut être formé à partir d'un tube présentant un diamètre constant qui, dans un premier temps, est dilaté par endroits de façon à obtenir les diamètres souhaités dans chacune des portions correspondant aux premier, deuxième et troisième éléments gonflables.

Dans un deuxième temps, le tube ainsi obtenu est conformé sous pression de façon à obtenir le profil final recherché (spires hélicoïdales).

La conformation précitée permet d'éviter l'apparition éventuelle de plicatures sur les spires lors du gonflage. De telles plicatures nuiraient en effet au bon déploiement de la structure gonflable 2 et à l'étanchéité de la cavité 9 au niveau des spires jointives 31 et 41 des premier et deuxième éléments gonflables 3, 4.

La structure gonflable 2 comprend également une membrane 8 fixée aux premier et deuxième éléments gonflables 3, 4 en définissant ainsi avec ces derniers, lorsque ceux-ci sont à l'état gonflé :
- d'une part, un manchon sensiblement cylindrique reliant entre elles de façon étanche les ouvertures centrales 21 desdits premier et deuxième éléments gonflables 3, 4 en formant ainsi un conduit de passage du fluide corporel et ;
- d'autre part, une cavité sensiblement annulaire et étanche 9 destinée à contenir une ou plusieurs substances thérapeutiquement actives et délimitée latéralement du côté proximal par la spire la plus distale du premier élément gonflable 3 et du côté distal par la spire la plus proximale de l'élément gonflable 4, et radialement extérieurement par la paroi interne 5 du conduit corporel 1, et intérieurement par la surface externe de la membrane 8.

Dans le cadre de la présente description et des revendications, on entend par "substance thérapeutiquement active" toute substance ou composition ayant une activité thérapeutique. Une telle substance peut être de forme variée comme par exemple une colle, un gel, des microsphères, des particules, des nanoparticules.

La membrane 8 est disposée de préférence au niveau du diamètre intérieur des spires jointives 31 et 41 des premier et deuxième éléments gonflables 3, 4 en y étant liée de préférence de façon irréversible, par exemple par collage, thermocollage, soudure ou trempage.

Comme on le comprend, la portion de membrane comprise entre les deux groupes de spires jointives 31 et 41 définit ainsi un tube ouvert à ses deux extrémités créant un passage pour l'écoulement du fluide corporel dans le sens représenté par la flèche F.

Par construction, ce conduit de passage du fluide corporel peut présenter un diamètre relativement important permettant la mise en place du cathéter sans interruption du flux de ce fluide.

A titre d'exemple, le diamètre de ce conduit peut être de 2,5mm pour une artère de 3,5mm, ce qui permet d'obtenir un débit du fluide corporel au travers du cathéter supérieur à 50% du débit normal en l'absence de cathéter.

Lors du gonflage du tube formant les premier, deuxième et troisième éléments gonflables 3, 4, 7 la structure gonflable 1 va se déployer et entraîner une tension de la membrane.

La membrane 8 est généralement conformée de telle sorte que le conduit central soit cylindrique lorsque celle-ci est sous tension après gonflage sous une pression prédéterminée (généralement de l'ordre de 6 bars) des premier, deuxième et troisième éléments gonflables 3, 4, 7.

La membrane aura de préférence un comportement élastique radialement pour pouvoir suivre la compliance des spires jointives 31, 41.

A cet effet, la membrane peut être réalisée en une matière telle qu'un silicone, un polyuréthane ou un polyamide, supportant un étirement radial.

Dans un mode de réalisation actuellement préféré, la membrane est réalisée en polyuréthane et peut supporter un étirement radial équivalant à deux fois son diamètre initial.

La membrane 8 est déployée et mise en forme sous l'effet du gonflage des premier, deuxième et troisième éléments gonflables 3, 4, 7 en passant ainsi d'un état relativement souple à un état semi-rigide créant une barrière entre la lumière de passage du fluide corporel et l'espace annulaire 9 précité, cet espace étant relativement étanche par rapport au milieu ambiant et pouvant donc servir de poche ou de cavité de diffusion pour la ou les substances thérapeutiquement actives utilisées.

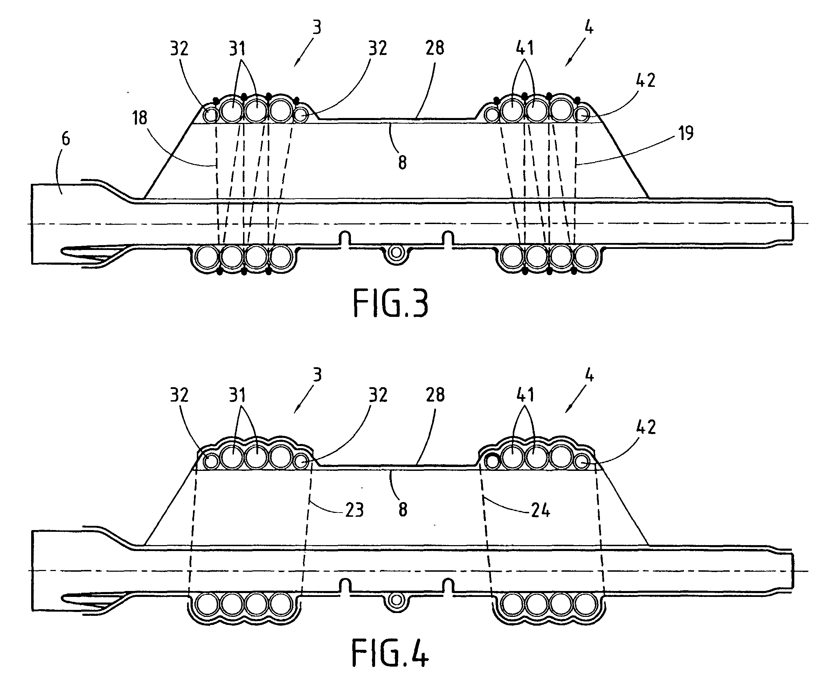

Dans le mode de réalisation représenté aux figures 3 et 4, la structure gonflable 2 comporte une deuxième membrane 28 disposée radialement et extérieurement relativement à la première membrane 8 pour envelopper les premier et deuxième éléments gonflables 3, 4.

La deuxième membrane 28 peut éventuellement se prolonger jusqu'au corps de cathéter pour envelopper partiellement celui-ci comme représenté aux figures 3 et 4.

La deuxième membrane sera liée par exemple par collage, thermocollage, soudure ou trempage aux premier et deuxième éléments gonflables 3, 4, et éventuellement au corps de cathéter ainsi qu'à la première membrane 8.

Cette conformation particulière permet d'obtenir une meilleure étanchéité de la cavité annulaire 9 ainsi qu'une meilleure résistance de la structure gonflable 2 longitudinalement et transversalement.

Il est encore à noter que la première membrane 8 est également fixée par exemple par collage, thermocollage, soudure ou trempage au troisième élément gonflable 7, ce dernier participant au maintien de la membrane en tension lors du gonflable de la structure gonflable 2 en évitant notamment le rapprochement relatif des premier et deuxième éléments gonflables 3, 4.

Le rôle du troisième élément gonflable est essentiel pour maintenir constante la section du conduit de passage du fluide corporel au cours de l'intervention quelle que soit la distance séparant les premier et deuxième éléments gonflables 3, 4.

Le cathéter conforme à la présente invention comporte un corps de cathéter généralement constitué par un élément tubulaire allongé flexible dont la longueur totale peut être d'environ 135cm et le diamètre variable de 0,70mm à 3mm en fonction de l'application désirée.

Avantageusement, ce diamètre sera d'environ 1mm dans le cas du traitement préventif de la resténose.

Le corps de cathéter est préférentiellement constitué de plusieurs parties de diamètres distincts réalisées dans des matières présentant des flexibilités différentes.

La partie proximale présente une longueur totale d'environ 110cm, et la partie distale une longueur totale d'environ 25cm.

L'extrémité distale représentée à la figure 1 et destinée à porter la structure gonflable est avantageusement étirée sur une longueur légèrement supérieure à la longueur de la portion du conduit corporel à traiter, par exemple d'environ 3cm dans ce cas particulier.

Le corps de cathéter peut être réalisé par exemple en une matière telle qu'un thermoplastique semi-rigide comme par exemple un polyéthylène haute densité, un polyamide, ou un copolymère de type PEBAX® commercialisé par la Société ATOCHEM.

Le corps de cathéter, et ceci constitue une caractéristique originale de l'invention, est disposé à l'intérieur de la structure gonflable précitée, c'est-à-dire à l'intérieur du conduit de passage du fluide corporel comme le montrent notamment les figure 2B et 2C.

Ceci a notamment pour conséquence que le corps de cathéter n'est pas au contact direct avec la paroi interne 5 du conduit corporel 1, notamment au niveau du site à traiter, ce qui constitue un avantage déterminant par rapport au cathéter de l'état de la technique décrit dans le document EP-0.526.102.

Selon l'invention, le corps de cathéter comporte un premier conduit interne 11 communiquant d'une part avec le tube constituant les premier, deuxième et troisième éléments gonflables 3, 4, 7 et d'autre part, avec un moyen non représenté permettant le gonflage et le dégonflage de ce tube.

Dans le mode de réalisation actuellement préféré, la communication entre le conduit interne 11 et le tube constituant les premier, deuxième et troisième éléments gonflables 3, 4, 7 est réalisée par l'intermédiaire de la portion de tube 32 définie précédemment.

Le corps de cathéter comprend en outre un second conduit interne 12 communiquant d'une part avec la cavité annulaire 9 précitée et d'autre part avec une source d'alimentation en fluide contenant une ou plusieurs substances pharmacologiquement actives.

Dans le mode de réalisation actuellement préféré, la communication entre le second conduit interne 12 du corps de cathéter et la cavité 9 précitée est réalisée par l'intermédiaire d'un ou plusieurs orifices de diffusion 13 réalisés dans le corps de cathéter et dans la membrane 8 de préférence sous la forme d'encoches s'étendant longitudinalement et présentant une longueur d'environ 2mm.

Dans le mode de réalisation représenté à la figure 1, il n'y a qu'un seul orifice de diffusion 13 réalisé sous la forme d'une encoche de 2mm de longueur et présentant un diamètre de 0,2mm.

La forme particulière de cette encoche permet une diffusion homogène de la substance pharmacologiquement active dans la cavité annulaire 9.

Il est à noter que grâce à cette forme particulière, la substance pharmacologiquement active diffuse au travers des orifices 13 sous forme de gouttes non traumatisantes pour la paroi du conduit morphologique, ce qui constitue également un avantage.

La paroi du conduit est donc progressivement atteinte par la substance pharmacologiquement active, le débit de diffusion et donc la pression à l'intérieur de la cavité annulaire 9 pouvant être ensuite augmentée sensiblement si nécessaire.

Selon une caractéristique particulière, la surface externe de la deuxième membrane 28 peut être poreuse dans la partie comprise entre les premier et deuxième éléments gonflables 3,4, permettant une diffusion encore plus homogène et encore moins traumatisante de la substance pharmacologiquement active à l'intérieur de la cavité annulaire 9.

Le corps de cathéter peut en outre comprendre un troisième conduit interne 12A indépendant du second conduit interne 12, et communiquant d'une part avec la cavité annulaire 9 précitée et d'autre part soit avec une source d'alimentation en fluide contenant éventuellement une ou plusieurs substances actives identiques ou différentes de la ou des substances actives susceptibles d'être véhiculées par le second conduit interne 12, soit avec un dispositif d'aspiration.

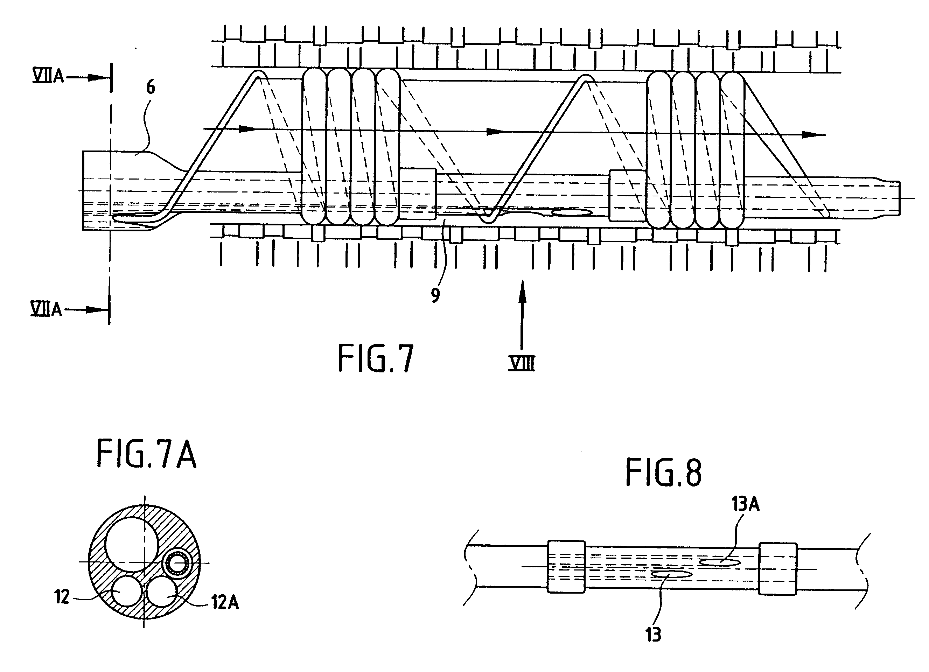

Dans le mode de réalisation actuellement préféré et représenté aux figures 7, 7A et 8, la communication entre le troisième conduit interne 12A du corps de cathéter et la cavité 9 précitée est réalisée par l'intermédiaire d'un ou plusieurs orifices de diffusion 13A présentant de préférence la même conformation que les orifices de diffusion 13 précités.

Le dispositif d'aspiration relié à l'éventuel troisième conduit interne 12A peut servir par exemple dans le cadre du traitement des anévrismes pour permettre d'évacuer le liquide contenu dans la poche de l'anévrisme, et dans ce cas le second conduit interne 12 permet de remplir ladite poche de l'anévrisme au moyen d'une substance thérapeutiquement active adéquate.

Le corps de cathéter peut en outre comprendre un quatrième conduit interne 14 constituant une voie de passage pour un fil guide non représenté ou toute autre sonde.

Le cathéter utilisé dans le cadre de la présente invention peut être de type "échange rapide", c'est-à-dire que son corps ne sera traversé par le fil guide que sur une faible portion de sa longueur correspondant sensiblement à la partie distale. Dans ce cas, le corps de cathéter en partie proximale peut comporter uniquement les premier et deuxième conduits internes précités.

Le cathéter peut être également du type connu sous la dénomination "over the wire", c'est-à-dire que son corps sera traversé par le guide sur toute sa longueur. Dans ce cas, il sera nécessaire de prévoir le troisième conduit précité constituant la voie de passage du guide.

Le cathéter peut également être pourvu en outre d'une âme à haut module élastique telle que définie dans le document WO 95/28197, s'étendant le long du corps de cathéter sensiblement jusqu'à l'extrémité distale afin de permettre une transmission optimale de la poussée exercée par le praticien au niveau de l'extrémité proximale du cathéter lors de son introduction dans le conduit corporel.

Pour permettre l'introduction et le retrait du cathéter conforme à la présente invention dans un conduit corporel, il est nécessaire que le corps de cathéter présente notamment au niveau de la partie distale portant la structure gonflable précitée, un diamètre réduit, lorsque cette dernière est à l'état dégonflé.

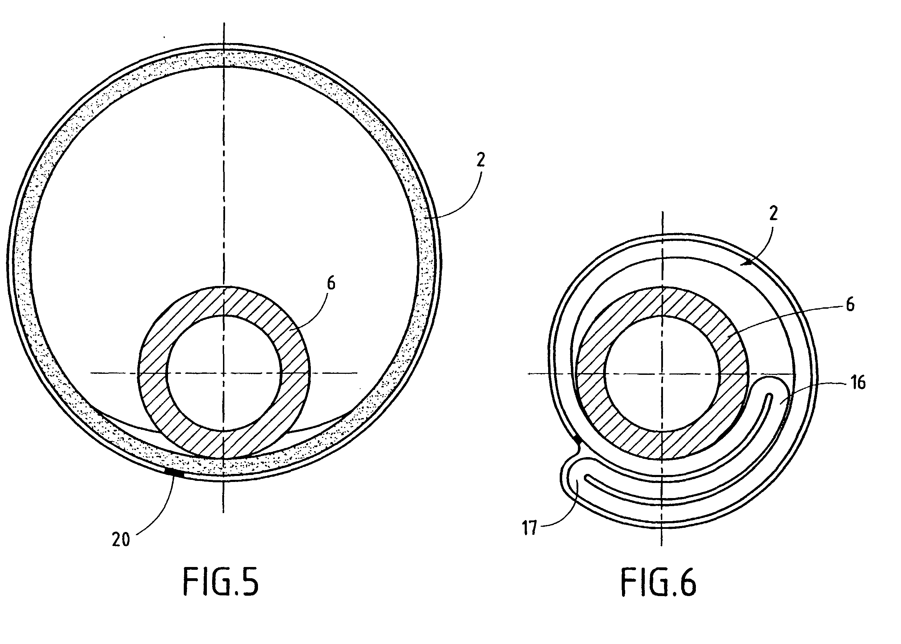

Pour faciliter le pliage de la structure gonflable autour du corps de cathéter et rendre ainsi plus aisée l'introduction de ce dernier dans le conduit corporel, la structure gonflable est préformée, par exemple dans la conformation représentée à la figure 6, ce préformage conférant en outre à la structure gonflable une "mémoire" facilitant son repliage et rendant ainsi plus aisé le retrait du cathéter après intervention.

Comme le montre la figure 6, la structure gonflable peut être ainsi formée par exemple sous pression pour présenter, vue en coupe transversale relativement au corps de cathéter, la forme générale d'une spirale refermée sur elle-même par deux boucles alternées 16, 17.

Pour faciliter encore le repliage de la structure gonflable 2, un cathéter conforme à la présente invention peut en outre être pourvu de moyens d'aide au repliage après dégonflage de la structure gonflable précitée.

Selon un premier mode de réalisation représenté à la figure 3, ces moyens d'assistance au repliage comprennent deux fils élastiques 18, 19, disposés respectivement autour des premier et deuxième éléments gonflables 3, 4, en y étant liés, de préférence selon une génératrice 20 (voir figure 5).

Cette liaison peut être réalisée par exemple par collage, thermocollage, soudure ou trempage.

Les fils élastiques précités peuvent être réalisés en une matière très élastique comme par exemple le latex, certains polyuréthanes ou silicones.

Selon un autre mode de réalisation représenté à la figure 4, les moyens d'assistance au repliage comprennent deux membranes élastiques 23, 24, sensiblement cylindriques, enveloppant respectivement les premier et deuxième éléments gonflables 3, 4, en y étant également liés de préférence selon une génératrice 20 (voir figure 5).

Comme on le comprend, les éléments d'aide au repliage vont agir après le dégonflage de la structure gonflable 2 en exerçant une force de rappel élastique facilitant le repliage de cette structure 2 dans sa position initiale telle que représentée à la figure 6.

Cette caractéristique particulière confère au cathéter de l'invention une grande sécurité lors de son introduction et de son retrait du conduit corporel.

La mise en oeuvre du cathéter conforme à la présente invention se déduit aisément de sa structure qui vient d'être décrite.

Préalablement à l'introduction du cathéter dans le conduit corporel, le conduit 12 du cathéter est rempli d'un sérum physiologique afin d'éliminer toutes les bulles d'air présentes dans ce conduit.

Le cathéter, pourvu de sa structure gonflable à l'état dégonflé et replié est introduit de façon traditionnelle par exemple au moyen d'un fil-guide dans un conduit morphologique tel qu'un vaisseau sanguin et positionné grâce à des marqueurs radio-opaques 10 (voir figure 1) à l'endroit du site destiné à être traité, par exemple le site d'une sténose dilatée par angioplastie.

Lorsque le cathéter est ainsi positionné, la structure gonflable 2 est alimenté par la voie de gonflage 11 jusqu'à une pression égale à environ une partie de la pression normale d'utilisation (par exemple 2 bars).

Lors de ce gonflage préliminaire, la structure gonflable 2 va se déployer et entraîner la mise en tension de la membrane 8.

La cavité annulaire 9 est alors alimentée par l'intermédiaire du conduit 12 par une faible quantité (de l'ordre de 1cm³)d'un fluide contenant une ou plusieurs substances actives afin de remplacer le sérum physiologique présent dans ledit conduit. La pression maximum au cours de cette opération est de 1 bar.

Ensuite, la structure gonflable est amenée progressivement à la pression d'étanchéité souhaitée qui peut être de l'ordre de 4 à 14 bars.

La substance thérapeutiquement active est alors introduite dans la cavité annulaire étanche 9 au débit souhaité.

De nombreuses substances thérapeutiquement actives peuvent être administrées dans le cadre de la présente invention comme par exemple des anti-thrombines, des inhibiteurs de l'enzyme de conversion, des vasodilatateurs, des agents lipidémients ou encore certains proto-oncogènes.

Le cathéter qui vient d'être décrit est particulièrement avantageux puisqu'il permet de diffuser très précisément les substances thérapeutiquement actives variées à des concentrations voulues pendant un temps prédéterminé.

En outre, la conformation particulière de ce cathéter permet de traiter en une seule opération l'intégralité de la surface du conduit corporel au niveau du site choisi.

Le cathéter qui vient d'être décrit peut également être utilisé pour isoler un segment de la paroi d'un conduit corporel, notamment pour traiter un anévrisme, pour aider une intervention chirurgicale sur ledit segment de paroi ainsi isolé, comme par exemple un pontage, ou encore pour réaliser une embolisation sélective.

Dans le cas particulier du traitement des anévrismes, le cathéter conforme à l'invention permet d'isoler la malformation du reste du flux sanguin, afin de pouvoir traiter celle-ci beaucoup plus efficacement et sûrement qu'avec les moyens existants. Le risque d'embollisation accidentelle est ainsi considérablement réduit.

## Revendications

1. Cathéter destiné à être introduit dans un conduit corporel où circule un fluide notamment pour y délivrer, dans une zone localisée, une substance thérapeutiquement active véhiculée par un second fluide, comprenant:
- un corps de cathéter (6) comportant un élément tubulaire allongé présentant une partie proximale et une partie distale ;
- une structure gonflable disposée au niveau de la partie distale dudit corps de cathéter et comportant :
- un premier élément gonflable (3) et un deuxième élément gonflable (4) qui, à l'état gonflé :
- sont axialement espacés l'un de l'autre ;
- portent chacun contre la paroi interne dudit conduit corporel ; et
- présentent chacun une ouverture sensiblement centrale (21);
- une membrane (8) fixée aux dits premier et deuxième éléments gonflables (3, 4) et définissant avec ces derniers, lorsque ceux-ci sont à l'état gonflé ;
- d'une part, un manchon sensiblement cylindrique reliant entre elles de façon étanche les ouvertures centrales desdits premier et deuxième éléments gonflables formant ainsi un conduit de passage du fluide corporel ; et
- d'autre part, une cavité (9) sensiblement étanche susceptible de contenir ladite substance thérapeutiquement active et délimitée par lesdits premier et deuxième éléments gonflables (3, 4), par la surface externe de ladite membrane (8) et par la surface interne du conduit corporel ;
- un troisième élément gonflable (7), disposé entre lesdits premier et deuxième éléments gonflables (3, 4) conformé pour maintenir à l'état gonflé ladite membrane en position ;
ledit corps de cathéter comportant un premier conduit interne (11) communiquant d'une part avec lesdits premier, deuxième et troisième éléments gonflables et d'autre part avec un moyen permettant le gonflage et le dégonflage de ces éléments ; un second conduit interne (12) communiquant d'une part avec la cavité précitée et d'autre part avec une source d'alimentation en fluide contenant ladite substance pharmacologiquement active, et éventuellement un troisième conduit interne (12A), indépendant dudit second conduit interne, communiquant d'une part avec la cavité précitée et d'autre part avec une source d'alimentation en fluide contenant éventuellement une substance thérapeutiquement active ou avec un dispositif d'aspiration, ledit corps de cathéter étant disposé à l'intérieur de l'enroulement dudit tube, dans le conduit central précité **caractérisé en ce que** lesdits premier (3), deuxième (4) et troisième (7) éléments gonflables sont réalisés en une seule pièce préformée constituée d'un tube enroulé de façon hélicoïdale, ledit troisième (7) élément étant constitué par une ou plusieurs spires de cet enroulement, le diamètre dudit tube au niveau de la portion constituant ledit troisième élément étant inférieur au diamètre dudit tube au niveau des portions de tube constituant respectivement lesdits premier et deuxième éléments et destinées à porter contre la paroi interne dudit conduit corporel.

2. Cathéter selon la revendication 1, **caractérisé en ce que** la portion de tube constituant respectivement les premier et deuxième éléments gonflables (3, 4) présente une configuration en hélice à pas variable définissant une première zone constituée d'au moins deux spires jointives (31, 41) destinées à porter de façon étanche contre la paroi interne du conduit corporel, et une deuxième zone prolongeant la première et s'étendant jusqu'au corps de cathéter, ladite deuxième zone formant au moins une portion d'hélice (32, 42) dont le pas est supérieur à celui de l'hélice formée par les spires de la première zone, le diamètre du tube au niveau de la deuxième zone étant de préférence inférieur au diamètre du tube au niveau de la première zone.

3. Cathéter selon la revendication 1 ou 2, **caractérisé en ce que** la structure gonflable (2) précitée est préformée, de préférence de façon à présenter, vue en coupe transversale relativement au corps de cathéter, la forme générale d'une spirale refermée sur elle-même par une série d'au moins deux boucles alternées (16, 17).

4. Cathéter selon l'une des revendications 1 à 3, **caractérisé en ce qu'**il comprend des moyens d'aide au repliage après dégonflage de la structure gonflable (2) précitée.

5. Cathéter selon la revendication 4, **caractérisé en ce que** les moyens d'aide au repliage précités comprennent deux fils élastiques (18, 19) disposés respectivement autour des premier et deuxième éléments gonflables (3, 4) en y étant liés de préférence selon une génératrice (20).

6. Cathéter selon la revendication 4, **caractérisé en ce que** les moyens d'aide au repliage comprennent deux membranes élastiques (23, 24) sensiblement cylindriques enveloppant respectivement les premier et deuxième éléments gonflables (3, 4) en y étant liés de préférence selon une génératrice (20).

7. Cathéter selon l'une des revendications 1 à 6, **caractérisé en ce que** la membrane (8) précitée est disposée au niveau du diamètre intérieur des spires jointives (31, 41) des premier et deuxième éléments gonflables (3, 4) en y étant liée de préférence de façon irréversible, par exemple par collage, thermocollage, soudure ou trempage.

8. Cathéter selon la revendication 7, **caractérisé en ce que** la membrane (8) précitée est réalisée en une matière supportant un étirement radial telle qu'en particulier un silicone, un polyuréthane ou un polyamide.

9. Cathéter selon l'une des revendications 1 à 8, **caractérisé en ce que** la structure gonflable (2) précitée comporte une deuxième membrane (28) disposée radialement et extérieurement relativement à la première membrane (8) et enveloppant les premier et deuxième éléments gonflables (3, 4) précités.

10. Cathéter selon l'une des revendications 1 à 9, **caractérisé en ce que** la communication entre le second conduit interne (12) du corps de cathéter et la cavité (9) précitée est réalisée par l'intermédiaire d'un ou plusieurs orifices de diffusion (13) se présentant sous la forme d'encoches longitudinales assurant une diffusion de la substance thérapeutiquement active sous forme de gouttes non traumatisantes pour la paroi du conduit corporel.

11. Cathéter selon l'une des revendications 1 à 10, **caractérisé en ce que** ledit corps de cathéter comporte un troisième conduit interne (12A) communiquant d'une part avec la cavité (9) précitée et d'autre part soit avec une source d'alimentation en fluide contenant éventuellement une substance thérapeutiquement active, soit avec un dispositif d'aspiration.

## Patentansprüche

1. Katheter, der bestimmt ist zum Einführen in einen Körperkanal, wo ein Fluid zirkuliert, insbesondere um dort in einer örtlich begrenzten Zone eine von einem zweiten Fluid transportierte therapeutisch wirksame Substanz abzugeben, umfassend:
- einen Katheterkörper (6) mit einem langgestreckten röhrenförmigen Element, das einen proximalen Teil und einen distalen Teil aufweist;
- eine aufpumpbare Struktur, die in Höhe des distalen Teils des Katheterkörpers angeordnet ist und aufweist:
- ein erstes aufpumpbares Element (3) und ein zweites aufpumpbares Element (4), die in aufgepumptem Zustand:
- axial im Abstand voneinander angeordnet sind;
- jeweils gegen die Innenwand des Körperkanals anliegen; und
- jeweils eine im Wesentlichen mittige Öffnung (21) aufweisen;
- eine Membran (8), die am ersten und zweiten aufpumpbaren Element (3, 4) befestigt ist und mit diesen letzteren, wenn sich diese im aufgepumpten Zustand befinden, bildet:
- einerseits eine im Wesentlichen zylindrische Manschette, die in dichter Weise die mittigen Öffnungen des ersten und zweiten aufpumpbaren Elements untereinander verbindet, womit ein Durchtrittskanal für das Körperfluid gebildet wird; und
- andererseits eine im Wesentlichen dichte Ausnehmung (9), die geeignet ist, die therapeutisch wirksame Substanz zu enthalten, und die von dem ersten und zweiten aufpumpbaren Element (3, 4), von der äußeren Oberfläche der Membran (8) und von der inneren Oberfläche des Körperkanals begrenzt wird;
- ein zwischen dem ersten und zweiten aufpumpbaren Element (3, 4) angeordnetes drittes aufpumpbares Element (7), das angepasst ist, um im aufgepumpten Zustand die Membran in Position zu halten;
wobei der Katheterkörper aufweist: einen ersten inneren Kanal (11), der einerseits mit dem ersten, zweiten und dritten aufpumpbaren Element und andererseits mit einer Einrichtung verbunden ist, die das Aufpumpen und das Entleeren dieser Elemente gestattet; einen zweiten inneren Kanal (12), der einerseits mit der vorgenannten Ausnehmung und andererseits mit einer Quelle zur Versorgung mit einem die pharmakologisch wirksame Substanz enthaltenden Fluid verbunden ist, und gegebenenfalls einen vom zweiten inneren Kanal unabhängigen dritten inneren Kanal (12A), der einerseits mit der vorgenannten Ausnehmung und andererseits mit einer Quelle zur Versorgung mit einem gegebenenfalls eine therapeutisch wirksame Substanz enthaltenden Fluid oder mit einer Absaugvorrichtung verbunden ist, wobei der Katheterkörper innerhalb von der Wicklung der Röhre in dem vorgenannten mittleren Kanal angeordnet ist, **dadurch gekennzeichnet, dass** das erste (3), zweite (4) und dritte (7) aufpumpbare Element in einem einzigen vorgeformten Teil hergestellt sind, das aus einer schraubenoder wendelförmig gewickelten Röhre besteht, wobei das dritte Element (7) von einer oder mehreren Windungen dieser Wicklung gebildet wird, wobei der Durchmesser der Röhre in Höhe des Teils, der das dritte Element bildet, kleiner ist als der Durchmesser der Röhre in Höhe der Röhrenteile, die das erste bzw. zweite Element bilden und dazu bestimmt sind, gegen die Innenwand des Körperkanals anzuliegen.

2. Katheter nach Anspruch 1, **dadurch gekennzeichnet, dass** der Röhrenteil, der das erste bzw. zweite aufpumpbare Element (3, 4) bildet, eine Gestalt in Form einer Wendel mit wechselnder Steigung aufweist, die eine erste Zone bildet, welche aus mindestens zwei aneinanderstoßenden Windungen (31, 41) besteht, die dazu bestimmt sind, in dichter Weise gegen die Innenwand des Körperkanals anzuliegen, sowie eine zweite Zone, welche die erste verlängert und sich bis zum Katheterkörper erstreckt,
wobei die zweite Zone mindestens einen Wendelteil (32, 42) bildet, dessen Steigung größer ist als diejenige der von den Windungen der ersten Zone gebildeten Wendel, wobei der Durchmesser der Röhre in Höhe der zweiten Zone vorzugsweise kleiner ist als der Durchmesser der Röhre in Höhe der ersten Zone.

3. Katheter nach Anspruch 1 oder 2, **dadurch gekennzeichnet, dass** die vorgenannte aufpumpbare Struktur (2) vorgeformt ist, vorzugsweise derart, dass sie, im Verhältnis zum Katheterkörper im Querschnitt gesehen, die allgemeine Form einer Spirale aufweist, die durch eine Reihe von mindestens zwei alternierenden Schlaufen oder Schlingen (16, 17) wieder auf sich selbst geschlossen ist.

4. Katheter nach einem der Ansprüche 1 bis 3, **dadurch gekennzeichnet, dass** er Einrichtungen zur Unterstützung des Wiederzusammenlegens nach einem Entleeren der vorgenannten aufpumpbaren Struktur (2) umfasst.

5. Katheter nach Anspruch 4, **dadurch gekennzeichnet, dass** die vorgenannten Einrichtungen zur Unterstützung des Wiederzusammenlegens zwei elastische Fäden (18, 19) umfassen, die um das erste bzw. zweite aufpumpbare Element (3, 4) herum angeordnet sind, wobei sie damit vorzugsweise entlang von einer Mantellinie (20) verbunden sind.

6. Katheter nach Anspruch 4, **dadurch gekennzeichnet, dass** die Einrichtungen zur Unterstützung des Wiederzusammenlegens zwei im Wesentlichen zylindrische elastische Membranen (23, 24) umfassen, die das erste bzw. zweite aufpumpbare Element (3, 4) umhüllen, wobei sie damit vorzugsweise entlang von einer Mantellinie (20) verbunden sind.

7. Katheter nach einem der Ansprüche 1 bis 6, **dadurch gekennzeichnet, dass** die vorgenannte Membran (8) in Höhe des Innendurchmessers der aneinanderstoßenden Windungen (31, 41) des ersten und zweiten aufpumpbaren Elements (3, 4) angeordnet ist, wobei sie damit vorzugsweise in irreversibler Weise verbunden ist, zum Beispiel durch Kleben, Heißsiegeln, Verschweißen oder Tauchbeschichten.

8. Katheter nach Anspruch 7, **dadurch gekennzeichnet, dass** die vorgenannte Membran (8) aus einem eine radiale Dehnung verkraftenden Material hergestellt ist, wie insbesondere aus einem Silikon, einem Polyurethan oder einem Polyamid.

9. Katheter nach einem der Ansprüche 1 bis 8, **dadurch gekennzeichnet, dass** die vorgenannte aufpumpbare Struktur (2) eine zweite Membran (28) aufweist, die im Verhältnis zur ersten Membran (8) radial und außerhalb angeordnet ist und das vorgenannte erste und zweite aufpumpbare Element (3, 4) umhüllt.

10. Katheter nach einem der Ansprüche 1 bis 9, **dadurch gekennzeichnet, dass** die Verbindung zwischen dem zweiten inneren Kanal (12) des Katheterkörpers und der vorgenannten Ausnehmung (9) über eine oder mehrere Ausbreitungsöffnungen (13) hergestellt wird, die sich in Form von Längseinschnitten darbieten, welche eine Ausbreitung der therapeutisch wirksamen Substanz in Form von Tropfen sicherstellen, die für die Wand des Körperkanals nicht traumatisierend sind.

11. Katheter nach einem der Ansprüche 1 bis 10, **dadurch gekennzeichnet, dass** der Katheterkörper einen dritten inneren Kanal (12A) aufweist, der einerseits mit der vorgenannten Ausnehmung (9) und andererseits entweder mit einer Quelle zur Versorgung mit einem gegebenenfalls eine therapeutisch wirksame Substanz enthaltenden Fluid oder mit einer Absaugvorrichtung verbunden ist.

## Claims

1. A catheter designed to be introduced into a body canal in which a fluid circulates, in particular to deliver, at a localised zone, a therapeutically active substance carried by a second fluid, comprising:
- a catheter body (6) comprising an elongate tubular member having a proximal part and a distal part;
- an inflatable structure disposed at the level of the distal part of said catheter body and comprising:
- a first inflatable member (3) and a second inflatable member (4) which, in the inflated state:
- are spaced axially relative to one another;
- each bear against the inner wall of said body canal; and
- each have a substantially central opening (21);
- a membrane (8) fixed to said first and second inflatable members (3, 4) and defining with the latter, when the latter are in the inflated state:
- on the one hand, a substantially cylindrical sleeve connecting together in fluid-tight manner the central openings of said first and second inflatable members, thereby forming a duct for passage of the body fluid; and
- on the other hand, a substantially fluid-tight cavity (9) capable of containing said therapeutically active substance and defined by said first and second inflatable members (3, 4), by the outer surface of said membrane (8) and by the inner surface of the body canal;
- a third inflatable member (7) disposed between said first and second inflatable members (3, 4) and designed to hold said membrane in position when in the inflated state;
said catheter body comprising a first inner duct (11) communicating on the one hand with said first, second and third inflatable members and on the other hand with a means allowing inflation and deflation of said members; and a second inner duct (12) communicating on the one hand with the above-mentioned cavity and on the other hand with a fluid supply source containing said pharmacologically active substance, and optionally a third inner duct (12A), independent of said second inner duct, communicating on the one hand with the above-mentioned cavity and on the other hand with a fluid supply source optionally containing a therapeutically active substance or with a suction device, said catheter body being disposed inside the winding of said tube, in the above-mentioned central duct, **characterised in that** said first (3), second (4) and third (7) inflatable members are made of a single preshaped element consisting of a helicoidally wound tube, said third (7) member consisting of one or more turns of said winding, the diameter of said tube at the level of the portion constituting said third member being smaller than the diameter of said tube at the level of the tube portions respectively constituting said first and second members and designed to bear against the inner wall of said body canal.

2. A catheter according to claim 1, **characterised in that** the tube portions respectively constituting the first and second inflatable members (3, 4) have a variable pitch helical configuration defining a first zone consisting of at least two contiguous turns (31, 41) designed to bear in fluid-tight manner against the inner wall of the body canal, and a second zone extending the first and reaching as far as the catheter body, said second zone forming at least one helix portion (32, 42) with a larger pitch than that of the helix formed by the turns of the first zone, the tube diameter at the level of the second zone preferably being smaller than the tube diameter at the level of the first zone.

3. A catheter according to claim 1 or claim 2, **characterised in that** the inflatable structure (2) is preferably preshaped in such a way as to present, when viewed in transverse section relative to the catheter body, the general form of a spiral closed on itself by a series of at least two alternating loops (16, 17).

4. A catheter according to one of claims 1 to 3, **characterised in that** it comprises means for assisting in refolding after deflation of the above-mentioned inflatable structure (2).

5. A catheter according to claim 4, **characterised in that** the above-mentioned means for assisting in refolding comprise two elastic threads (18, 19) disposed respectively about the first and second inflatable members (3, 4) and being connected thereto preferably in accordance with a generating line (20).

6. A catheter according to claim 4, **characterised in that** the means for assisting in refolding comprise two substantially cylindrical resilient membranes (23, 24) respectively enveloping the first and second inflatable members (3, 4) and being connected thereto preferably in accordance with a generating line (20).

7. A catheter according to one of claims 1 to 6, **characterised in that** the above-mentioned membrane (8) is disposed at the level of the internal diameter of the contiguous turns (31, 41) of the first and second inflatable members (3, 4), being preferably irreversibly connected thereto for example by adhesion, thermal bonding, welding or heat treatment.

8. A catheter according to claim 7, **characterised in that** the above-mentioned membrane (8) is made of a material which withstands radial stretching, such as in particular a silicone, a polyurethane or a polyamide.

9. A catheter according to one of claims 1 to 8, **characterised in that** the above-mentioned inflatable structure (2) comprises a second membrane (28) disposed radially and externally relative to the first membrane (8) and enveloping the above-mentioned first and second inflatable members (3, 4).

10. A catheter according to one of claims 1 to 9, **characterised in that** communication between the second inner duct (12) of the catheter body and the above-mentioned cavity (9) is achieved through the intermediary of one or more diffusion orifices (13) in the form of longitudinal notches ensuring diffusion of the therapeutically active substance in the form of drops which are non-traumatising for the wall of said body canal.

11. A catheter according to one of claims 1 to 10, **characterised in that** said catheter body comprises a third inner duct (12A) communicating on the one hand with the above-mentioned cavity (9) and on the other hand either with a fluid supply source optionally containing a therapeutically active substance or with a suction device.
